(19) Europäisches Patentamt / European Patent Office / Office européen des brevets

(11) **EP 4 359 766 B1**

(12) **EUROPÄISCHE PATENTSCHRIFT**

(45) Veröffentlichungstag und Bekanntmachung des Hinweises auf die Patenterteilung:
**30.07.2025 Patentblatt 2025/31**

(21) Anmeldenummer: **22740753.3**

(22) Anmeldetag: **24.06.2022**

(51) Internationale Patentklassifikation (IPC):
**G01N 21/3504** (2014.01) **G01S 7/481** (2006.01)
**G01S 17/86** (2020.01) **G01S 17/88** (2006.01)
G01N 21/3563 (2014.01) G01N 21/17 (2006.01)

(52) Gemeinsame Patentklassifikation (CPC):
**G01N 21/3504; G01S 7/4813; G01S 7/4818;
G01S 17/86; G01S 17/88;** G01N 21/3563;
G01N 2021/178

(86) Internationale Anmeldenummer:
**PCT/AT2022/060218**

(87) Internationale Veröffentlichungsnummer:
**WO 2022/266692 (29.12.2022 Gazette 2022/52)**

(54) **VORRICHTUNG UND VERFAHREN ZUR KONZENTRATIONSBESTIMMUNG EINES STOFFES IN EINEM MESSVOLUMEN**

APPARATUS AND METHOD FOR DETERMINING A CONCENTRATION OF A SUBSTANCE IN A MEASURING VOLUME

DISPOSITIF ET PROCÉDÉ POUR DÉTERMINER UNE CONCENTRATION D'UNE SUBSTANCE DANS UN VOLUME DE MESURE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.06.2021 AT 505322021**

(43) Veröffentlichungstag der Anmeldung:
**01.05.2024 Patentblatt 2024/18**

(73) Patentinhaber: **AVL List GmbH
8020 Graz (AT)**

(72) Erfinder:
• PEIN, Andreas
8341 Paldau (AT)
• STEINER, Gerald
8053 Graz (AT)
• ARAR, Mario
8020 Graz (AT)

(74) Vertreter: **Patentanwälte Pinter & Weiss OG
Prinz-Eugen-Straße 70
1040 Wien (AT)**

(56) Entgegenhaltungen:
WO-A1-01/48456 CN-U- 208 224 072
US-A- 3 677 652 US-A- 4 924 095
US-A1- 2011 038 507

• PUNDT IRENE ED - TURNER CHARLOTTA ET AL: "DOAS tomography for the localisation and quantification of anthropogenic air pollution", ANALYTICAL AND BIOANALYTICAL CHEMISTRY, SPRINGER BERLIN HEIDELBERG, BERLIN/HEIDELBERG, vol. 385, no. 1, 21 February 2006 (2006-02-21), pages 18 - 21, XP037836292, ISSN: 1618-2642, [retrieved on 20060221], DOI: 10.1007/S00216-005-0205-4

**Beschreibung**

Die gegenständliche Erfindung betrifft eine Vorrichtung zur Konzentrationsbestimmung zumindest eines gasförmigen oder festen Stoffes in zumindest einem Messvolumen an einer stationären Messstelle, wobei zumindest eine Messeinheit vorgesehen ist, einen Lichtstrahl mit vorgegebener Lichtintensität durch das Messvolumen abzustrahlen, und zumindest ein Detektor vorgesehen ist, um den Lichtstrahl nach dem Durchtritt durch das Messvolumen zu erfassen, wobei der zumindest eine Detektor ausgebildet ist, eine Abnahme der Lichtintensität aufgrund des zumindest einen gasförmigen oder festen Stoffes im Messvolumen zu ermitteln.

Die Erfindung betrifft ferner ein entsprechendes Verfahren zur Konzentrationsbestimmung zumindest eines gasförmigen oder festen Stoffes in einer Abgaswolke in zumindest einem Messvolumen.

Emissionen von Stoffen in Abgasen, speziell im Individualverkehr, sind durch die steigende Anzahl speziell an Fahrzeugen nicht nur im Zuge der Klimaerwärmung, sondern auch im Zuge der gesundheitlichen Belastung für den Menschen durch Stickoxide, teilverbrannte Kraftstoffbestandteile und Feinstaubpartikel ein vieldiskutiertes Thema. Entwicklungen der letzten Jahrzehnte zielten auf der einen Seite im Zuge des verpflichtenden Katalysatoreinbaus in Ottomotoren auf Vermeidung des Ausstoßes von teilverbrannten Verbindungen, und bei Katalysatoren in Dieselmotoren auf der anderen Seite auf Vermeidung des Ausstoßes von Stickoxiden. Die zulässigen Werte von emittierten Stoffen sind oftmals über nationale und supranationale Normen bestimmt.

Nichtsdestotrotz sind heutzutage noch immer Fahrzeuge im öffentlichen Raum im Einsatz, welche zwar zum Zeitpunkt ihrer Zulassung die gesetzlichen Normen zur Abgasreduktion erfüllt haben, bei der Verwendung über einen längeren Zeitraum aber als hohe Emittenten angesehen werden. Der Grund dafür kann beispielsweise das fehlende Nachrüsten eines Katalysators sein oder fehlende Wartung, wenn beispielsweise bei einem Dieselkatalysator das Nachfüllen von Harnstoff unterbleibt und eine ordnungsgemäße Funktion eines SCR (selektive katalytische Reduktion) Katalysators nicht mehr gegeben ist. Unter anderem kann das auch auf fehlendes Wissen bezüglich der (Nicht)-Funktionalität von eben diesen Bestandteilen während der Fahrt zurückzuführen sein.

Abgasmessungen sind großteils auf Systeme limitiert, welche im Fahrzeug selbst, beispielsweise im oder nach dem Auspuff, Stoffe im Abgas, wie gasförmige Stoffe oder Partikel messen. Diese Systeme sind jedoch auf eine geringe Anzahl an Testfahrzeugen beschränkt und können daher kein repräsentatives Abbild von einer Vielzahl an verschiedenen Fahrzeugen im Realbetrieb geben. Abgasmessungen im Rahmen der regelmäßigen Überprüfung des Fahrzeugs in einer Werkstatt ist ebensowenig repräsentativ, weil solche Überprüfungen nur in großen Zeitabständen durchgeführt werden. Daher wird versucht, Abgasmessungen von Fahrzeugen im Realbetrieb im öffentlichen Raum zu ermöglichen. Dieses sogenannte "Remote Sensing", auch im Sinne von "real driving emissions" (RDE) Messungen erfolgt an einer stationären Messstelle und kann beispielsweise an vorteilhaft vorinstallierter Infrastruktur, wie Mautstellen, Straßenlaternen, Brücken, oder auch Gebäudefassaden in der Stadt und ähnlichem angebracht werden. Das könnte beispielsweise dazu benutzt werden, Fahrzeughalter von Fahrzeugen mit hohen Emissionen zu benachrichtigen und/oder verpflichtende Wartungen vorzusehen. Man muss jedoch bei der Aufstellung der Geräte auf eine geeignete Messstelle achten, um repräsentative Ergebnisse zu erhalten. Generell sollten Kreuzungsbereiche mit Ampeln und damit potentiellem Stillstand der Fahrzeuge vermieden werden. Weiters hat sich gezeigt, dass eine leichte Steigung der Straße an der Messstelle dafür geeignet ist, eine positive Motorlast zu erzeugen.

Bei Remote Sensing wird oftmals eine Lichtquelle verwendet, welche eine charakteristische Wellenlänge oder Wellenlängenbereich(e) emittiert, um einen gasförmigen Stoff, wie Kohlenmonoxid oder Stickoxide, nachzuweisen. Ein Detektor ermöglicht beispielsweise eine Messung der Abschwächung des Lichts, das durch die Abgaswolke gesendet wird. Eine derartige Konzentrationsbestimmung mittels Absorptionsspektroskopie ist aus US 4,924,095 A bekannt. US 4,924,095 A beschreibt ein Analysegerät für die Überwachung der Abgasemissionen von Kraftfahrzeugen an einer stationären Messstelle, bei dem eine ebene Anordnung von Gasanalysatoren mit entsprechenden Strahlungsquellen und Detektoren die Intensitätsänderung von Strahlen bestimmter Wellenlängen beim Durchgang durch einen Querschnitt einer Abgasfahne messen, um die Konzentration von Schadstoffen im Abgas zu bestimmen. Eine dreidimensionale Matrix von Strahlen wird so positioniert, dass die Abgasfahne durch die Matrix hindurchgeht. Dabei kann die Anordnung eine relative Änderung einer Schadstoffmenge pro Volumeneinheit bestimmen, die von einem Kraftfahrzeug ausgestoßen wird, das die Anordnung passiert.

Es kann aber auch vorgesehen sein, Partikel, wie Rußpartikel, als Stoff zu messen. Das kann dann beispielsweise über Lichtstreuung oder über die Messung der Abschwächung des Rückstrahls im Verhältnis zum eingestrahlten Licht realisiert werden. Die verlässliche Messung solcher Stoffe in Abgaswolken kann jedoch zu unterschiedlichen Schwierigkeiten führen. Einerseits sind die Emissionen von Stoffen von unterschiedlichen Motoren oder auch anderen Energiesystemen, wie Brennstoffzellen, durchwegs unterschiedlich und müssen mit dem gleichen System messbar sein. Weiters schwankt der zu messende Konzentrationsbereich sehr stark und ist von der zu messenden Fahrzeugklasse (z.B. LKW vs. Motorrad) abhängig. Speziell geringe Konzentrationen bereiten bei der Auswertung Probleme. Auch Unterschiede in der Betriebstemperatur eines Motors können Unterschiede in den zu messenden Stoffen nach sich ziehen.

Damit können zwar Absorptionswerte bestimmt werden, diese sind aber wegen der oben genannten Unterschiedlichkeit schwer bis gar nicht vergleichbar. Insbesondere kann damit keine Konzentration eines gasförmigen oder festen Stoffes in der Abgaswolke bestimmt werden.

Heute verfügbare Systeme im Stand der Technik erfassen gasförmige oder feste Stoffe als optische Masse (OM), welche im Regelfall auf eine konstante Referenzkonzentration eines zweiten Stoffes in der Abgaswolke bezogen werden kann, beispielsweise Kohlendioxid

($CO_2$). Ein solcher Referenzwert kann für manche Abgaswolken repräsentiere Werte liefern. Beispielsweise kann für Abgaswolken aus Benzin-Verbrennungsmotoren ein solcher Referenzwert verwendet werden, um damit auch verschiedene OMs von unterschiedlichen Stoffen der Abgaswolken verlässlich zu bestimmen. Wenn beispielsweise die Absorption, eines Stoffes sich in verschiedenen Abgaswolken unterscheidet, ist die Absorption des Referenzwertes proportional und kann in Verhältnis gesetzt werden. Folglich kann über die bekannte Konzentration des Referenzwertes und der Absorption des Referenzwertes auf die Konzentration des Stoffes rückgerechnet werden. Diese Art der Berechnung kann für Verbrennungsmotoren mit Benzin relativ zuverlässig funktionieren, da durch die stöchiometrische Verbrennung der Konzentrationswert relativ konstant ist. Bei Fahrzeugen, die mit Diesel betrieben werden, kann diese Art der Konzentrationsberechnung allerdings nicht verwendet werden, da durch den nichtstöchiometrischen Verbrennungsvorgang sehr starke Schwankungen in der Referenzkonzentration, beispielsweise eine $CO_2$-Konzentration, möglich sind. Dieses Vorgehen ist daher unzuverlässig.

CN 103712929 A1 beschreibt ein System zur Lokalisierung und Messung einer Abgaswolke mittels optischer Kamera, Infrarotkamera und spektroskopischer Einheit. Dabei misst die optische Kamera die Fahrzeuggeschwindigkeit und ermittelt das Kennzeichen. Die Infrarotkamera lokalisiert die Position der Abgaswolke und die spektroskopische Messeinheit wird ausgerichtet, um die Abgaswolke zu messen. Die Offenbarung verbessert zwar das Lokalisieren der Abgaswolke, ermöglicht aber nicht, einen Konzentrationswert eines Abgasbestandteils zu bestimmen.

EP 3 789 755 offenbart eine Messmethode für Abgaswolken mittels multispektraler Analyse mit einer Mehrzahl an Infrarotkameras. Die IR-Kameras sind über oder neben der Straße angeordnet und könne mittels geeigneter Filter beispielsweise Kohlenmonoxid und/oder Kohlendioxid messen. Die Anordnung hat den Nachteil, dass keine spektroskopische Auswertung genutzt wird und daher die Möglichkeit der Messungen von Abgaskomponenten limitiert ist. Weiters hat es den Nachteil, dass derartige Methoden sehr geringe Sensivitäten

besitzen und weiters auch nicht in der Lage sind, absolute Konzentrationen direkt zu messen.

Die vorliegende Erfindung ist in den Ansprüchen 1 und 14 definiert.

Es ist eine Aufgabe der gegenständlichen Erfindung, bei einer Remote Sensing Messung zuverlässige Konzentrationsmessungen eines gasförmigen oder festen Stoffes in einem Messvolumen zu ermöglichen.

Diese Aufgabe wird durch eine eingangs genannte Vorrichtung erfindungsgemäß dadurch gelöst, dass in der Vorrichtung eine Bildeinheit vorgesehen ist, um bei teilweisem oder vollständigem Vorhandensein einer Abgaswolke im Messvolumen zumindest einen Teil der Abgaswolke aufzunehmen, dass in der Vorrichtung eine Auswerteeinheit vorgesehen ist, um aus zumindest einer aufgenommenen Abbildung des zumindest einen Teils der Abgaswolke eine Gesamtdurchtrittsstrecke des Lichtstrahls durch die Abgaswolke im Messvolumen zu bestimmen, und dass die Auswerteeinheit vorgesehen ist, aus der ermittelten Abnahme der Lichtintensität und der Gesamtdurchtrittsstrecke eine Konzentration des gasförmigen oder festen Stoffes im Messvolumen zu ermitteln.

Die erfindungsgemäße Konzentrationsbestimmung aus der Gesamtdurchtrittsstrecke des Lichtstrahls durch eine Abgaswolke im Messvolumen und aus einer ermittelten Abnahme der Lichtintensität des Lichtstrahls aufgrund des zumindest einen gasförmigen oder festen Stoffes, erlaubt die Konzentration des Stoffes direkt, zuverlässig und genau zu ermitteln. Diese Ermittlung der Konzentration ist insbesondere auch unabhängig von der Ausdehnung oder Zusammensetzung der Abgaswolke.

In einer vorteilhaften Ausgestaltung ist die zumindest eine Messeinheit beabstandet von zumindest einer ersten Spiegelungseinheit angeordnet, wobei das Messvolumen zwischen der zumindest einen Messeinheit und der zumindest einen ersten Spiegelungseinheit ausgebildet ist und die zumindest eine Messeinheit vorgesehen ist, einen Lichtstrahl in Richtung der zumindest einen Spiegelungseinheit abzustrahlen, und wobei die zumindest eine erste Spiegelungseinheit vorgesehen ist, den Lichtstrahl nach Durchtritt durch das Messvolumen zu reflektieren und zu zumindest einem Detektor zu senden. Eine solche Ausgestaltung kann die Anordnung des Detektors in der Vorrichtung vereinfachen, weil der Detektor nicht mehr zwingend gegenüber der Messeinheit angeordnet sein muss. Damit wird die Verwendung der Vorrichtung flexibler. Dabei ist es insbesondere vorteilhaft, wenn der an der Spiegelungseinheit reflektierte Lichtstrahl das Messvolumen vor Erreichen des Detektors zumindest ein weiteres Mal durchdringt. Aufgrund des zweimaligen Durchtritts des Lichtstrahls durch das Messvolumen kann die Sensitivität der Konzentrationsmessung erhöht werden und es können geringere Konzentrationen des gasförmigen oder festen Stoffes gemessen werden.

Günstigerweise ist dabei die Gesamtdurchtrittsstrecke die Summe der Teildurchtrittsstrecken aller Durchtritte des Lichtstrahls durch die Abgaswolke.

In einer Variante der Erfindung ist in der Messeinheit eine Multiplexereinheit vorgesehen, die eine Mehrzahl an Lichtstrahlen erzeugt, und die Messeinheit strahlt die Mehrzahl an Lichtstrahlen an unterschiedlichen Stellen durch das Messvolumen ab und zumindest ein Detektor jeden der Mehrzahl an Lichtstrahlen, vorzugsweise nach einer Reflexion an einer Spiegelungseinheit, erfasst. Dadurch kann auf einfache Weise eine ortsaufgelöste Konzentrationsmessung im Messvolumen erzielt werden. Damit kann die Konzentration des gasförmigen oder festen Stoffes an verschiedenen Stellen einer Abgaswolke im Messvolumen ermittelt werden.

Wenn zumindest zwei der Mehrzahl an Lichtstrahlen in der Messeinheit über jeweils einen optischen Pfad geführt sind, wobei vorzugsweise die optischen Pfade zumindest teilweise unterschiedliche optische Weglängen aufweisen, kann die Erfassung dieser Lichtstrahlen an einem Detektor vereinfacht werden, weil die Lichtstrahlen aufgrund der sich ergebenden unterschiedlichen Laufzeiten zeitversetzt am Detektor ankommen.

Um eine zweidimensionale Auflösung des Messvolumens zu erzielen, strahlt die Messeinheit in einer Variante der Erfindung zumindest einen ersten Lichtstrahl der Mehrzahl an Lichtstrahlen in einer ersten Richtung durch das Messvolumen ab und die Messeinheit strahlt einen zweiten Lichtstrahl der Mehrzahl an Lichtstrahlen in einer zweiten Richtung durch das Messvolumen ab, wobei die erste Richtung unterschiedlich zur zweiten Richtung ist. Damit kann die Konzentration des gasförmigen oder festen Stoffes in unterschiedlichen Dimensionen erfasst werden. Wenn das mit einer örtlich aufgelösten Konzentrationsmessung kombiniert wird, ist eine sehr genaue Rekonstruktion einer Konzentrationsverteilung in der Abgaswolke möglich.

Die Anzahl der Durchtritte des Lichtstrahls durch das Messvolumen kann weiter erhöht werden, wenn in der Vorrichtung eine zweite Spiegelungseinheit vorgesehen ist, die beabstandet und gegenüberliegend von der zumindest einen ersten Spiegelungseinheit angeordnet ist und die Messeinheit den Lichtstrahl in einem von einer Normalen auf eine erste Spiegelungsebene der ersten Spiegelungseinheit abweichenden Winkel abstrahlt, wobei die erste Spiegelungseinheit den von der Messeinheit abgestrahlten Lichtstrahl zur gegenüberliegenden zweiten Spiegelungseinheit reflektiert und die gegenüberliegende zweite Spiegelungseinheit den Lichtstrahl wieder zur ersten Spiegelungseinheit zurückreflektiert, wobei der zumindest eine Detektor vorgesehen ist, den Lichtstrahl nach einer Mehrzahl solcher Reflexionen zu erfassen. Besonders vorteilhaft kann dabei der Winkel des Lichtstrahls verstellbar sein, um die Anzahl der Reflexionen anwendungsbezogen wählen und einstellen zu können. In einer derartigen Variante ist in der Messeinheit eine Positionierungsoptikeinheit vorgesehen, mit der der Winkel des Lichtstrahls verstellbar ist.

In der Vorrichtung können auch mehrere Messeinheiten vorgesehen sein, wobei zumindest zwei der Messeinheiten einen Lichtstrahl durch unterschiedliche Messvolumina abstrahlen. In einer weiteren Variante sind in der Vorrichtung mehrere Messeinheiten vorgesehen, wobei zumindest zwei der Messeinheiten einen Lichtstrahl mit unterschiedlichen Richtungen durch dasselbe Messvolumen abstrahlen. Damit kann die Vorrichtung sehr flexibel und einfach auf verschiedene Anwendungen angepasst werden.

Vorzugsweise weist die Bildeinheit zumindest eine Kamera und/oder zumindest eine Lidareinheit auf.

Um empfindliche Spiegelungseinheiten vor Verschmutzung oder Beschädigung zu schützen ist vorteilhafterweise eine Schutzfolie austauschbar über die Spiegelungseinheit angeordnet. Damit können notwendige Wartungsintervalle reduziert werden.

Die Aufgabe der Erfindung wird außerdem durch ein eingangs genanntes Verfahren gelöst, bei dem die zumindest eine Messeinheit einen Lichtstrahl mit einer vorgegebenen Lichtintensität durch die Abgaswolke im Messvolumen abstrahlt der Lichtstrahl nach Durchtritt durch die Abgaswolke von einem Detektor erfasst wird und der Detektor eine Abnahme der Lichtintensität des Lichtstrahls aufgrund des zumindest einen gasförmigen oder festen Stoffes ermittelt, wobei erfindungsgemäß zumindest ein Teil der Abgaswolke im Messvolumen von einer Bildeinheit aufgenommen wird und aus der aufgenommenen Abbildung des zumindest einen Teils der Abgaswolke eine Gesamtdurchtrittsstrecke des Lichtstrahls durch die Abgaswolke im Messvolumen bestimmt wird, und dass aus der ermittelten Abnahme der Lichtintensität und der Gesamtdurchtrittsstrecke eine Konzentration des gasförmigen oder festen Stoffes im Messvolumen ermittelt wird.

Die gegenständliche Erfindung wird nachfolgend unter Bezugnahme auf die Figuren 1 bis 6 näher erläutert, die beispielhaft, schematisch und nicht einschränkend vorteilhafte Ausgestaltungen der Erfindung zeigen. Dabei zeigt

Fig.1 das Prinzip der entfernten Abgasmessung (remote sensing),

Fig.2 eine erfindungsgemäße Ausführungsform der Konzentrationsbestimmung in einer Abgaswolke,

Fig.3 eine vorteilhafte Ausführungsform einer Messeinheit,

Fig.4 eine Ausgestaltung der Vorrichtung mit einer Lidareinheit als Bildeinheit,

Fig.5 eine Ausgestaltung der Vorrichtung mit einem mehrfachen Durchtritt des Lichtstrahls durch die Abgaswolke, und

Fig.6 einen Ausführungsform für den Schutz einer Spiegelungseinheit.

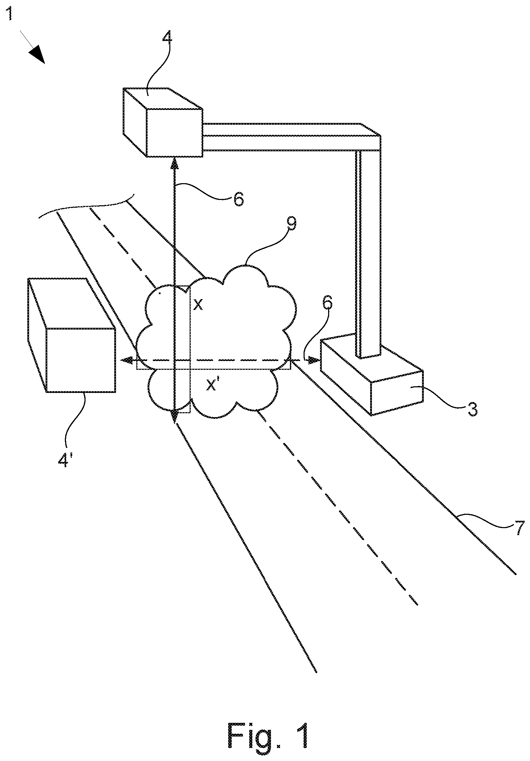

Fig. 1 zeigt eine Vorrichtung 1 gemäß dem Stand der Technik zur Messung eines gasförmigen oder festen Stoffes in einer Abgaswolke 9, welche von einer Emissionsquelle, wie einem Fahrzeug im öffentlichen Raum, ausgestoßen wird. In der Abgaswolke 9 können verschiedenste gasförmige und feste (z.B. Partikel) Bestandteile vorkommen. Die Stoffe in der Abgaswolke 9 können von jeder Art von Emissionsquelle, beispielsweise auf einer Oberfläche 7, stammen, beispielsweise von einem Fahrzeug wie einem Personenkraftwagen (PKW), Lastkraftwagen (LKW), aber auch einem einspurigen Fahrzeug wie einem Motorrad, Motorfahrrad und ähnliches, welche einen Verbrennungsmotor aufweisen. Emissionen einer anderen Emissionsquelle, wie Brennstoffzellen, welche im Regelfall nur Wasserdampf und keine Schadstoffe emittieren, können ebenfalls über eine solche Vorrichtung 1 gemessen werden. Die Erfassung solcher Emissionsquellen kann hilfreich sein, um beispielsweise den Anteil an Fahrzeugen mit niedrigen oder hohen Emissionswerten im Straßenverkehr zu ermitteln. Die Messung kann an einer Oberfläche 7 erfolgen, beispielsweise einer Straße, vorteilhafterweise in einem gewissen Abstand über einer Oberfläche 7. Es ist aber auch vorstellbar, dass die Vorrichtung 1 seitlich einer Abgaswolke 9 angeordnet ist und die Messung parallel zu einer Oberfläche 7 erfolgt, oder die Vorrichtung 1 kann auch in der Oberfläche 7 selbst verbaut sein. Auch Kombinationen von Messungen von mehreren Seiten sind vorstellbar.

Die Vorrichtung 1 kann eine Abgaswolke 9 beispielsweise auch an anderen Orten abseits einer Oberfläche 7 messen. Es ist vorstellbar, dass die Vorrichtung 1 eine Abgaswolke 9 eines Flugzeugs bei Start oder Landung auf einer Landepiste auf einem Flughafen misst. Auch ist es denkbar, dass eine Abgaswolke 9 von einem Schiff, beispielsweise in einem Hafenbecken oder in einer Schleuse gemessen wird.

Neben der Anwendung in der Automobilindustrie, sind auch andere Anwendungen, in denen Abgaswolken 9 mit gasförmigen oder festen Stoffen als Emissionen entstehen, denkbar, beispielsweise in der Prozessindustrie. Hierbei können beispielsweise Emissionen in Schornsteinen gemessen werden, die Durchmesser von einigen Metern aufweisen können. Das Messvolumen $V_M$ wäre dabei im Schornstein ausgebildet.

Die Erfindung ist nicht auf oben genannte Anwendungen limitiert, sondern alle Einsatzmöglichkeiten, die sich dem Fachmann erschließen, sind vorstellbar. Die Abgaswolke 9 muss aber nicht zwingend von einem Fahrzeug stammen, sondern kann prinzipiell von jeglicher Emissionsquelle herrühren. Ein Beispiel ist eine Abgaswolke aus einem Industrieprozess, die beispielsweise an einem Schornstein abgegeben wird.

Die Stoffe in einer Abgaswolke 9 können gasförmige Stoffe, wie Kohlendioxid ($CO_2$), Kohlenmonoxid (CO), Stickoxide ($NO_X$), Schwefeldioxid ($SO_2$), gasförmige polyzyklische aromatische Kohlenwasserstoffe (PAK) und ähnliches sein. Es ist aber auch denkbar, feste Stoffe in einer Abgaswolke 9, wie Rußpartikel, zu messen. Die Stoffe und deren Konzentrationen in der Abgaswolke 9 eines Fahrzeugs sind üblicherweise abhängig vom Typ des Kraftstoffes, vom Verbrennungsmotor, vom Betriebszustand des Verbrennungsmotors, und vom Status eines Katalysators oder Abgasnachbehandlungssystems (sofern vorhanden). Beispielsweise emittiert ein Verbrennungsmotor, der noch nicht auf Betriebstemperatur ist, oftmals eine höhere Konzentration an teilverbrannten Stoffen, wie polyzyklische aromatische Kohlenwasserstoffe, als bei normaler Betriebstemperatur. Ebenso werden bei unterschiedlichen Betriebszuständen (z.B. gegeben durch aktuelle Drehzahl und aktuelles Drehmoment) unterschiedliche Stoffe emittiert.

Erfindungsgemäß soll die Konzentration eines solchen gasförmigen oder festen Stoffes in der Abgaswolke 9 gemessen werden.

Je nach Anordnung der Messeinheit 4 der Vorrichtung 1 kann ein Lichtstrahl 6 die Abgaswolke 9 auf verschiedenen Durchtrittsstrecken x durchdringen. Der Lichtstrahl 6 wird nach Durchtritt durch die Abgaswolke 9 in einem Detektor 3 erfasst und ausgewertet. Beispielsweise können bei unterschiedlichen Anordnungen, wie in Fig. 1 gegeben, die Durchtrittsstrecke x des Lichtstrahls 6, welcher normal auf die Oberfläche 7 ist, unterschiedlich zur anderen Durchtrittsstrecke x' eines anderen Lichtstrahls 6, welcher parallel zur Oberfläche 7 verläuft, sein und daher abhängig von der Anordnung der Messeinheiten 4, 4' relativ zur Abgaswolke 9 sein. Das kann für die nominell gleiche Konzentration eines Stoffes zu unterschiedlichen Messergebnissen für den Stoff in der Abgaswolke 9 führen, weil eine Absorption bei längerer Durchtrittsstrecke x, x' im Regelfall höher ist.

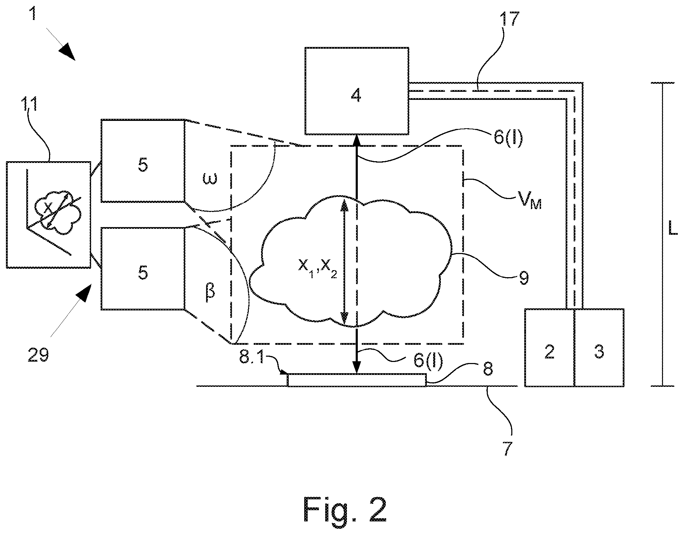

Fig. 2 zeigt eine erfindungsgemäße Ausführungsform der Vorrichtung 1, mit der eine Konzentration eines gasförmigen oder festen Stoffes in einer Abgaswolke 9 zuverlässig gemessen werden kann. In der Vorrichtung 1 ist in dieser Ausführung zumindest eine Lichtquelle 2 vorgesehen, die Licht mit einer vorgegebenen Lichtintensität erzeugt. Die Lichtquelle 2 kann beispielsweise monochromatisches Licht abgeben, beispielsweise ein Laserlicht, welches eine definierte Wellenlänge mit vorgegebener Lichtintensität aufweist. Insbesondere können Quanten-Kaskaden-Laser (QCL) verwendet werden, es sind aber auch andere Typen und Kombinationen von Lichtquellen und Lasern vorstellbar, um verschiedene Wellenlängenbereiche abzudecken. Es ist auch denkbar, dass die Lichtquelle 2 eine polychromatisch emittierende Lampe aufweist, wie eine Lampe im Ultraviolett- (UV) oder auch im Infrarotbereich (IR). Ebenfalls ist ein Monochromator in der Lichtquelle 2 denkbar, um gezielt Wellenlängen zu selektieren. Die Lichtquelle 2 erzeugt einen Primärlichtstrahl 17 mit einer bestimmten Lichtintensität und zumindest einer Wellenlänge. Der Primärlichtstrahl 17 kann beispielsweise über einen Lichtleiter, wie ein Glasfaserkabel, ein Spiegelsystem oder ein anderes geeignetes optisches System, zu zumindest einer Messeinheit 4 geleitet werden. Die Lichtquelle 2 kann aber auch in der Messeinheit 4 integriert sein und in der Messeinheit 4 den Primärlichtstrahl 17 erzeugen.

Die Messeinheit 4 erzeugt aus dem Primärlichtstrahl 17 der Lichtquelle 2 einen Lichtstrahl 6 mit zumindest einer definierten Wellenlänge und einer definierten Lichtintensität I, der von der Messeinheit 4 abgestrahlt wird. Vorzugsweise entsprechen die Wellenlänge und die Lichtintensität I des Lichtstrahls 6 der Wellenlänge und der Lichtintensität des Primärlichtstrahls 17. Die Messeinheit kann die Lichteigenschaften des Primärlichtstrahls 17 (z.B. Wellenlänge oder Lichtintensität) aber auch verändern, beispielsweise mittels eines Monochromators oder eines Strahlteilers, um den Lichtstrahl 6 zu erzeugen. Der Lichtstrahl 6 kann dabei auch gepulst, z.B. in Form von einzelnen Lichtpaketen, erzeugt werden.

Zur Durchführung der Konzentrationsmessung strahlt die Messeinheit 4 den Lichtstrahl 6 direkt oder indirekt in Richtung des Messvolumens $V_M$ ab, sodass der Lichtstrahl 6 das Messvolumen $V_M$ und eine darin teilweise oder vollständig befindliche Abgaswolke 9 durchdringt. Der Lichtstrahl 6 wird nach dem Durchtritt durch die Abgaswolke 9 im Messvolumen $V_M$ von einem Detektor 3 erfasst.

In der Ausgestaltung der Erfindung gemäß Fig.2 ist die Messeinheit 4 in einem Abstand L von einer Spiegelungseinheit 8 mit einer Spiegelungsebene 8.1, beispielsweise auf der Oberfläche 7, angeordnet. Die Spiegelungsebene 8.1 der Spiegelungseinheit 8 ist dabei der Messeinheit 4 zugewandt angeordnet. Dabei bildet sich zwischen der Messeinheit 4 und der Spiegelungseinheit 8 das Messvolumen $V_M$ aus, in dem sich in bestimmungsgemäßer Verwendung der Vorrichtung 1 eine zu messende Abgaswolke 9 teilweise oder vollständig befinden kann. An der Spiegelungseinheit 8, bzw. an der Spiegelungsebene 8.1, wird ein einfallender Lichtstrahl reflektiert.

Der Abstand L kann abhängig von der Anwendung sein, beispielsweise von den durchfahrenden Fahrzeugen, aber auch von der Richtung der Messung. Der Abstand L kann beispielsweise geringer sein, wenn eine Messung parallel zur Oberfläche 7 durchgeführt wird, und kann höher sein, wenn normal auf die Oberfläche 7 gemessen wird. Jedenfalls kann der Abstand L anwendungsbezogen passend gewählt werden.

Die Orientierung und Ausrichtung der Spiegelungseinheit 8 kann beliebig und anwendungsbezogen gewählt sein. Die Spiegelungseinheit 8 kann beispielsweise parallel zur Ebene der Oberfläche 7, normal zur Ebene der Oberfläche 7, oder auch in einem beliebigen Winkel zur Ebene der Oberfläche 7 montiert sein. Vorteilhafterweise kann die Spiegelungseinheit 8 auch in einer Oberfläche 7 integriert sein und mittels einer geeigneten Beschichtung oder eines Verbaus vor Schäden und Verschmutzung, beispielsweise durch Fahrzeuge, welche über die Oberfläche 7 fahren, geschützt werden.

Die Anordnung der Spiegelungseinheit 8 und die Orientierung des von der Messeinheit 4 abgestrahlten Lichtstrahls 6 ist jedenfalls so gewählt, dass der Lichtstrahl 6 das Messvolumen $V_M$ durchdringt und an der Spiegelungseinheit 8 auftrifft. Der Lichtstrahl 6 muss dabei aber nicht direkt auf die Spiegelungseinheit 8 gerichtet sein, sondern kann auch über ein optisches System, beispielsweise eine Spiegelanordnung, in Richtung der Spiegelungseinheit 8 gelenkt werden.

Die genauen Dimensionen des Messvolumens $V_M$ können vorgegeben sein oder von einem Anwender gewählt werden und beispielsweise abhängig von der erwartenden Ausdehnung einer Abgaswolke 9 sein. Das Messvolumen $V_M$ kann beispielsweise auch kleiner als eine erwartete Abgaswolke 9 gewählt sein, um eine Messung von Vermengungen verschiedener Abgaswolken 9 in einem Messvolumen $V_M$ von unterschiedlichen Emissionsquellen zu verhindern.

Zur Durchführung der Konzentrationsmessung mit einer Vorrichtung 1 in der Ausführungsform nach Fig.2 strahlt die Messeinheit 4 den Lichtstrahl 6 direkt oder indirekt in Richtung der Spiegelungseinheit 8 ab. Der ausgestrahlte Lichtstrahl 6 durchdringt dabei das Messvolumen $V_M$ und wird an der Spiegelungseinheit 8 reflektiert. Der reflektierte Lichtstrahl 6 wird zu zumindest einem Detektor 3 geleitet, der den reflektierten Lichtstrahl 6 erfasst. Der reflektierte Lichtstrahl 6 kann dabei das Messvolumen $V_M$, je nach Ausgestaltung der Reflexion, ein zweites Mal durchdringen. Damit durchdringt der Lichtstrahl 6 das Messvolumen $V_M$ und eine darin teilweise oder vollständig befindliche Abgaswolke 9 zumindest einmal. Vorzugsweise wird der reflektierte Lichtstrahl 6 an der Spiegelungseinheit 8 so reflektiert, dass auch der reflektierte Lichtstrahl 6 das Messvolumen $V_M$ und eine darin befindliche Abgaswolke 9 durchdringt. Durch das mehrmalige Durchdringen der Abgaswolke 9 durch den Lichtstrahl 6 kann die Sensitivität der Konzentrationsmessung erhöht werden, weil das Licht entlang des Strahlenganges mehrmals von den gasförmige oder festen Stoffen im Messvolumen $V_M$ beeinflusst wird und damit deutlichere Messsignale möglich sind.

Der Detektor 3 kann in örtlicher Nähe zu der Lichtquelle 2, beispielsweise in einem gemeinsamen Gehäuse mit der Lichtquelle 2, angeordnet sein, er kann aber beispielsweise auch in der Messeinheit 4 oder an einer beliebigen anderen Stelle der Vorrichtung 1 angeordnet sein. In der Vorrichtung 1 nach Fig.2 ist der Detektor 3 mit der Lichtquelle 2 in einem Gehäuse verbaut und der reflektierte Lichtstrahl 6 wird entlang desselben optischen Weges wie der Primärlichtstrahl 17 geleitet.

Der Detektor 3 misst erfindungsgemäß eine Abschwächung der Lichtintensität I des erfassten Lichtstrahls 6 aufgrund eines gasförmigen oder festen Stoffes im Messvolumen $V_M$. Diese Abschwächung der Lichtintensität I kann auf eine Referenzmessung bezogen werden, die bei Abwesenheit von Stoffen im Messvolumen $V_M$ durchgeführt wird und zu einer Referenz-Licht-

intensität $I_0$ führt. Damit kann beispielsweise eine Absorption $1-(I/I_0)$, kurz als A bezeichnet, oder Transmission $I/I_0$ einer bestimmten Wellenlänge, oder auch mehrerer unterschiedlicher Wellenlängen, berechnet werden. Die Transmission und die Absorption A können ineinander umgerechnet werden und können als äquivalent betrachtet werden.

Für feste Stoffe im Messvolumen $V_M$ kann ein Lichtintensitätsverlust durch eine Lichtstreuung, wie eine Vorwärts- oder Seitwärtsstreuung, verursacht sein. Der Detektor 3 kann beispielsweise auch eine Lichtstreuung an festen Partikeln im Messvolumen $V_M$ erfassen und daher eine Lichtintensitätsabnahme aufgrund von Streuung messen. Damit kann auch die Größe von festen Partikeln im Detektor 3 abgeschätzt werden. Auch ist es möglich, ein Spektrum über verschiedene Wellenlängen aufzunehmen, und eine spektroskopische Auswertung durchzuführen, beispielsweise in Form von multivarianten Analysen oder Fingerprinting eines Stoffes. Damit können auch mehrere verschiedene Stoffe gleichzeitig gemessen werden. Das kann auch über geeignete optische Filter oder über Monochromatoren ermöglicht werden.

Die Abnahme der Lichtintensität I des vom Detektor 3 erfassten Lichtstrahls 6 ist abhängig von der Gesamtdurchtrittsstrecke x des Lichtstrahls 6 durch eine Abgaswolke 9 im Messvolumen $V_M$ und kann auch je nach Anordnung der Messeinheit 4 und/oder der Spiegelungseinheit 8 unterschiedlich sein. Auch unterschiedliche Größen der Abgaswolke 9, abhängig vom Fahrzeug, können zu unterschiedlichen Messwerten am Detektor 3 führen.

Die Messung einer Absorption eines gasförmigen oder festen Stoffes ist bekanntermaßen frequenzabhängig und sollte daher am oder in der Nähe des Absorptionsmaximum stattfinden, um ein verlässliches Ergebnis zu erhalten. Beispielsweise hat $CO_2$ charakteristische Vibrationsschwingungen bei einer Wellenzahl (Kehrwert der Wellenlänge) von $1388\ cm^{-1}$ (asymmetrische Streckschwingung) und bei $667\ cm^{-1}$ (Biegeschwingung). Laut Lambert-Beer'schen Gesetz ist die Absorption A abhängig von der Gesamtdurchtrittsstrecke x, der Konzentration c und einem Absorptionskoeffizienten k (als bekannter Stoffparameter). Dieser Zusammenhang kann über die Formel

$$\ln \frac{I}{I_0} = k * x * c$$

angegeben werden. Um eine verlässliche Konzentration c eines Stoffes zu bestimmen, ist daher Kenntnis über die Gesamtdurchtrittsstrecke x und der Absorption A erforderlich. Eine Absorption A (oder gleichwertig eine Abnahme der Lichtintensität) kann über den Detektor 3 ermittelt werden. Die Gesamtdurchtrittsstrecke x ist aber abhängig von der Ausdehnung der Abgaswolke 9 im Messvolumen $V_M$ und dem Strahlengang des Lichts durch die Abgaswolke 9 und ist üblicherweise nicht bekannt.

Für eine verlässliche Konzentrationsmessung unterschiedlichster Abgaswolken 9 ist es daher vorteilhaft, Kenntnis über die Gesamtdurchtrittsstrecke x des Lichts durch die Abgaswolke 9 zu haben, da man dadurch nicht auf die Eingang erwähnte Berechnung über eine optische Masse mit den angeführten Beschränkungen zurückgreifen muss. Die Gesamtdurchtrittsstrecke x hängt vom Strahlengang des Lichtstrahls 6 ab und damit davon, wie oft und an welcher Stelle der Lichtstrahl 6 die Abgaswolke 9 durchdringt.

Zum Erfassen der Gesamtdurchtrittsstrecke x ist in der Vorrichtung 1 erfindungsgemäß eine Bildeinheit 29 vorgesehen, um zumindest einen Teil des Messvolumens $V_M$ aufzunehmen, vorzugsweise aus unterschiedlichen Richtungen (z.B. Winkeln $\omega$, $\beta$). Die Bildeinheit 29 erzeugt zumindest eine Abbildung eines Teils einer Abgaswolke 9 im Messvolumen $V_M$, vorzugsweise mehrere Abbildungen aus unterschiedlichen Richtungen, die in einer Auswerteeinheit 11 verarbeitet wird. Die Auswerteeinheit 11 kann nun aus der erhaltenen zumindest einen Abbildung die Gesamtdurchtrittsstrecke x des Lichtstrahls 6 durch die Abgaswolke 9 ermitteln. Dazu kann die Auswerteeinheit 11 zumindest einen Teil eines Abbilds einer Abgaswolke 9 im Messvolumen $V_M$ rekonstruieren. Aus dem Abbild des Teils der Abgaswolke 9 kann anhand des bekannten Strahlengangs des Lichts (der durch die Anordnung der Spiegelungseinheit 8 und der Orientierung des Lichtstrahls 6 festgelegt ist) durch das Messvolumen $V_M$ die Gesamtdurchtrittsstrecke x durch die Abgaswolke 9 ermittelt werden.

Die Gesamtdurchtrittsstrecke x umfasst zumindest die Teildurchtrittsstrecke x1 des Lichtstrahls 6 durch die Abgaswolke 9. Wird der Lichtstrahl 6 reflektiert und ein weiteres Mal durch die Abgaswolke 9 gelenkt, dann kommt noch die Teildurchtrittsstrecke x2 des reflektierten Lichtstrahl 6 hinzu. Die Gesamtdurchtrittsstrecke x ist damit die Summe der einzelnen Teildurchtrittsstrecken des Lichtstrahls 6 durch die Abgaswolke 9.

Beispielsweise kann anhand der bekannten Abmessungen des Messvolumens $V_M$ auf die Abmessungen des zumindest einen, rekonstruierten Teils der Abgaswolke 9 im Messvolumen $V_M$ und damit auf eine Teildurchtrittsstrecke x1, x2 rückgerechnet werden.

Bei einer Anordnung wie in Fig.2, bei der der Lichtstrahl 6 im Wesentlichen normal auf die Spiegelungsebene 8.1 der Spiegelungseinheit 8 gelenkt wird, sind die Teildurchtrittsstrecken x1, x2 des abgestrahlten Lichtstrahls 6 und des reflektierten Lichtstrahls 6 gleich, weil der Lichtstrahl 6 in die entgegengesetzte Richtung reflektiert wird. Es kann daher ausreichend sein, nur die Teildurchtrittsstrecke x1 des abgestrahlten Lichtstrahls 6 oder die Teildurchtrittsstrecke x2 des reflektierten Lichtstrahls 6 zu ermitteln und diese zu verdoppeln, um die Gesamtdurchtrittsstrecke x zu erhalten.

Zur Ermittlung der Teildurchtrittsstrecken x1, x2, bzw. der daraus abgeleiteten Gesamtdurchtrittsstrecke x, kann aus den mit der Bildeinheit 29 aufgenommenen Abbildungen eine 2D-Projektion der Abgaswolke 9 in der Ebene des Lichtstrahls 6 und/oder des reflektierten Lichtstrahls 6 erzeugt werden und so die Teildurchtrittsstrecken x1, x2 direkt ermittelt werden. Vorteilhafterweise erstellt die Auswerteinheit 11 eine räumliche Rekonstruktion der Abgaswolke 9, wofür Abbildungen der Abgaswolke 9 aus unterschiedlichen Richtungen erforderlich sind. Diese Rekonstruktion kann beispielsweise auch abhängig von einer Laufvariable, wie der Zeit, sein. So kann beispielsweise eine zeitabhängige Ausdehnung einer Abgaswolke 9 ermittelt werden.

In einer vorteilhaften Ausführungsform kann die Auswerteinheit 11 Daten über Außentemperatur und Luftfeuchte erhalten. Je nach Außentemperatur und Luftfeuchte kann es zu Unterschieden in der Auswertung und Rekonstruktion einer Abgaswolke 9 kommen. Beispielsweise sind Temperaturunterschiede im Sommer zwischen Umgebung und Abgaswolke 9 weniger stark ausgeprägt als im Winter. Das kann dazu führen, dass eine ermittelte Teildurchtrittsstrecke x1, x2 saisonabhängig Unterschiede aufweist. Um diese Fehlerquelle zu vermeiden oder zumindest einzugrenzen, kann ein Korrekturfaktor für die Berechnung der Rekonstruktion abhängig von Außentemperatur und Luftfeuchte vorgesehen sein. Damit kann die Auswerteinheit 11 eine verlässlichere Berechnung der Gesamtdurchtrittsstrecke x unabhängig von den Umgebungsbedingungen durchführen.

Die Auswerteinheit 11, üblicherweise ein Computer mit entsprechender Auswertesoftware, und kann ebenfalls Daten zur Absorption A (bzw. der Lichtintensität des erfassten Lichtstrahls 6) vom zumindest einen Detektor 3 erhalten. Eine Referenz-Lichtintensität I0 kann in der Auswerteeinheit 11 gespeichert sein und kann als bekannt vorausgesetzt werden. Die Auswerteeinheit 11 verwendet die Gesamtdurchtrittsstrecke x und die Abnahme der Lichtintensität, bzw. eine Absorption A, um die Konzentration c eines gasförmigen oder festen Stoffes zu ermitteln, beispielsweise nach dem oben angeführten Lambert-Beer'schen Gesetz. In einer vorteilhaften Ausführungsform können auch verschiedene Absorptionen A bei verschiedenen Wellenlängen des Lichtstrahls 6 genutzt werden, um Konzentrationen c verschiedener gasförmiger oder fester Stoffe zu ermitteln. Das kann hintereinander bei jeweils einer Wellenlänge erfolgen oder auch in Form einer spektroskopischen Auswertung bei mehreren Wellenlängen gleichzeitig.

Die Bildeinheit 29 kann in Form von zumindest einer Kamera 5, vorzugsweise mehrere Kameras 5 (wie in Fig.2 dargestellt) ausgeführt sein. Auch eine Ausführung der Bildeinheit 29 mit einer oder mehreren Lidareinheiten, einer oder mehreren Radareinheiten oder Kombinationen derartiger Einheiten bzw. mit Kameras ist denkbar. Daneben sind weitere Ausführungen einer Bildeinheit 29 möglich.

Die Bildeinheit 29, beispielsweise die zumindest eine Kamera 5, kann beispielsweise an der Messeinheit 4 angeordnet sein. In einer Variante ist die Bildeinheit 29 auf einer separaten Vorrichtung installiert, oder bestehende Infrastruktur im Bereich der Vorrichtung 1, wie Brücken, Häuser, Straßenlaternen oder ähnliches, werden zur Anordnung genutzt. Bei einer Mehrzahl an Messvolumina $V_M$ kann die Bildeinheit 29 auch so angeordnet sein, dass sie beispielsweise mehrere Messvolumina $V_M$ gleichzeitig erfassen kann. So kann die Anzahl der benötigten Bildeinheiten 29 geringgehalten werden.

In einer Ausführung der Bildeinheit 29 mit mehreren Kameras 5 sind diese an verschiedenen Orten installiert, um das Messvolumen $V_M$ aus verschiedenen Richtungen $\omega$, $\beta$ aufzunehmen. Bei der Aufnahme des Messvolumens $V_M$ aus unterschiedlichen Richtungen kann die Rekonstruktion der Abgaswolke 9 erleichtert oder verbessert werden.

Die zumindest eine Kamera 5 kann Abbildungen des Messvolumens $V_M$ aufnehmen und damit auch eine im Messvolumen $V_M$ vorhandene Abgaswolke 9 aufnehmen. Es ist aber auch möglich, dass die Kamera 5 zusätzlich Metadaten eines Fahrzeugs aufzeichnet, wie Größe, Typ oder auch das Kennzeichen. Bei Verwendung einer Kamera 5 als Bildeinheit 29 kann beispielsweise verfügbare Bildbearbeitungssoftware verwendet werden, um die Abgaswolke 9 oder einen Teil davon aus den Abbildungen zu rekonstruieren.

Die zumindest eine Kamera 5 kann beispielsweise eine Infrarotkamera sein, welche Wärmebilder der vorhandenen Abgaswolke im Messvolumen $V_M$ aufnimmt. So kann auch die Wärmeverteilung in der Abgaswolke 9 erfasst werden, welche einen Einfluss auf die gasförmigen oder festen Stoffe oder den Absorptionskoeffizienten k haben kann. Es kann aufgrund der Temperaturunterschiede zu Konvektion und Diffusionsphänomenen kommen, die eine Verteilung von Stoffen über die Zeit bewirken. Auch können einzelne Konzentrationen c von Stoffen abhängig von der Temperatur sein, da manche Reaktionen nur bei höherer Temperatur ablaufen oder temperaturabhängig sind.

Die zumindest eine Kamera 5 kann aber auch beispielsweise im ultravioletten (UV) oder sichtbaren (VIS) Bereich arbeiten, oder auch in beiden Bereichen (UV/VIS Kameras). UV oder VIS ist höher-energetische Strahlung als IR und regt Elektronen-Übergange in Molekülen an und kann für die Messung vorteilhafter sein.

In einer Ausführungsform kann die zumindest eine Kamera 5 als multi- und hyperspektrale Kamera ausgeführt sein. Dabei werden statt der klassischen einfachen Aufnahme in einem einfachen Spektralbereich eine hohe Anzahl an Spektralbändern benutzt. Das kann vorteilhaft sein, um eine deutlich höhere Farbqualität und Farbunterschiede zu erkennen, da jeder Pixel bereits ein vollständiges Farbspektrum enthält. In einer solche Kamera 5 kommt beispielsweise die Snapshot-Mosaiktechnik zur Anwendung.

Die Messstelle, insbesondere das Messvolumen $V_M$, ist während der Messung mit der Vorrichtung 1 stationär, es wird somit mit der Vorrichtung 1 eine stationäre Messung an einer bestimmten stationären Messstelle realisiert. Damit kann sich die Abgaswolke 9 während der Messung relativ zum Messvolumen $V_M$ bewegen oder verändern, die Vorrichtung 1, konkret die einzelnen Einheiten der Vorrichtung 1, bleiben dabei aber an der Messstelle ortsfest angeordnet. Insbesondere bleiben die zumindest eine Lichtquelle 2, eine Messeinheit 4, ein Detektor 3, eine Spiegelungseinheit 8, eine Bildeinheit 29 während der Messung ortsfest. Falls in der Vorrichtung 1 noch weitere Einheiten vorhanden sind, wie beispielsweise eine Multiplexereinheit 10, so sind auch diese während der Messung ortsfest.

Die erfindungsgemäße Vorrichtung 1 ist damit insbesondere keine Messeinheit, die in einem bewegten Fahrzeug zur Abgasmessung verbaut ist und sich während der Messung mit dem Fahrzeug mitbewegt.

Die Vorrichtung 1 ist aber nur während der Messung zwingend stationär angeordnet. Zwischen zwei Messungen kann die Vorrichtung 1 oder können Teile davon auch bewegt werden. Im Zuge von Remote-Sensing-Messungen kann es beispielsweise erforderlich sein, dass die Vorrichtung 1 zwischen verschiedenen Messstellen wechselt, beispielsweise um damit einen größeren Teil von in einem größeren Gebiet (z.B. einer Stadt) fahrenden Fahrzeugen zu erfassen. In diesem Zusammenhang kann auch vorgesehen sein, die Vorrichtung 1 teilweise oder vollständig auf einem Anhänger zu montieren, um die Vorrichtung 1 zwischen den Messstellen mobil wechseln zu können. Der Hauptteil der Vorrichtung 1, insbesondere eine Lichtquelle 2, eine Messeinheit 4, ein Detektor 3 und eine Bildeinheit 29, kann auf einem ausfahrbaren Gestell am Anhänger vormontiert sein. An der Messstelle anbringbare Teile der Vorrichtung 1, wie beispielsweise die Spiegelungseinheit 8 wird dann an der Messstelle in geeigneter Weise angeordnet. Während der Messung sind aber wieder alle Einheiten stationär.

Es kann vorkommen, dass sich verschiedene Abgaswolken 9 aus hinter- oder nebeneinander befindlichen Emissionsquellen, wie Fahrzeugen, im Messvolumen $V_M$ vermengen. Dann kann die Messung dementsprechend angepasst werden, beispielsweise über Positionierung oder Ausrichtung der Bildeinheit 29, insbesondere der zumindest einen Kamera 5.

In einer Variante ist eine Mehrzahl an Messeinheiten 4 in der erfindungsgemäßen Vorrichtung 1 vorgesehen, auch mit unterschiedlichen Abständen L zu einer jeweils zugeordneten Spiegelungseinheit 8. Es können auch mehrere Spiegelungseinheiten 8 vorgesehen sein, beispielsweise für jede Messeinheit 4 eine eigene oder jeweils eine für verschiedene Gruppen an Messeinheiten. Mehrere Messeinheiten 4 sind vorteilhaft, um ortsaufgelöste Messungen einer Abgaswolke 9 in einem Messvolumen $V_M$ zu realisieren, oder auch um mehrere Abgaswolken 9 in verschiedenen Messvolumina $V_M$ gleichzeitig zu vermessen. Hierzu kann ein Primärlichtstrahl 17 einer Lichtquelle 2 bedarfsweise auch mit optischen Systemen entsprechend aufgeteilt werden, beispielsweise mittels Strahlenteiler, Umlenkeinheiten, Spiegeln, Multiplexer usw., um jede der Messeinheiten 4 mit Licht zu versorgen. Es kann aber auch in jeder Messeinheit 4, oder in einigen davon, eine Lichtquelle 2 zur Erzeugung eines Primärlichtstrahls 17 vorgesehen sein.

Bei einer mehrfachen Messung einer Abgaswolke 9 kann ein Durchschnittswert der Konzentration eines gasförmigen oder festen Stoffes in der Abgaswolke 9 ermittelt werden. Es ist aber auch möglich, eine ortsaufgelöste Messung der Konzentration in der Abgaswolke 9 zu ermitteln. Bei entsprechender Anordnung der Messeinheiten 4 und Spiegelungseinheiten 8 kann auch eine zweidimensionale örtliche Auflösung der Konzentration eines gasförmigen oder festen Stoffes in der Abgaswolke 9 ermittelt werden. Hierfür könnten beispielsweise eine erste Gruppe von Lichtstrahlen 6 die Abgaswolke in einer ersten Richtung durchdringen und eine zweite Gruppe von Lichtstrahlen 6 die Abgaswolke in einer zweiten, von der ersten unterschiedlichen Richtung, beispielsweise normal auf die erste Richtung.

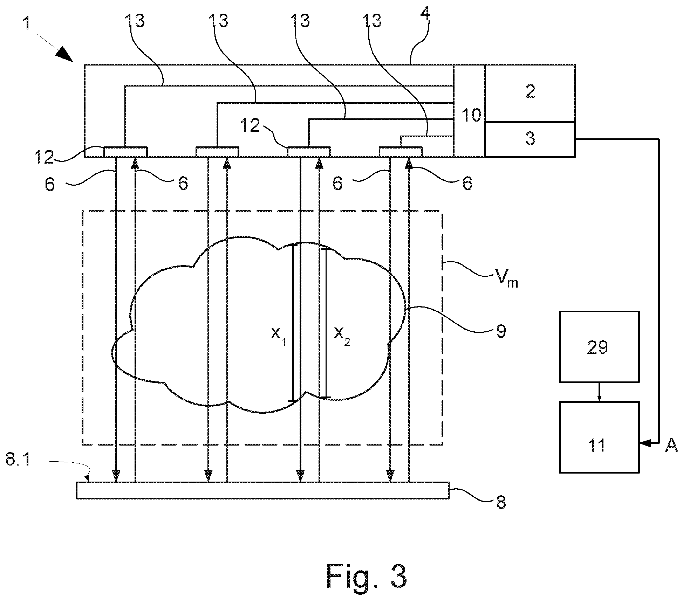

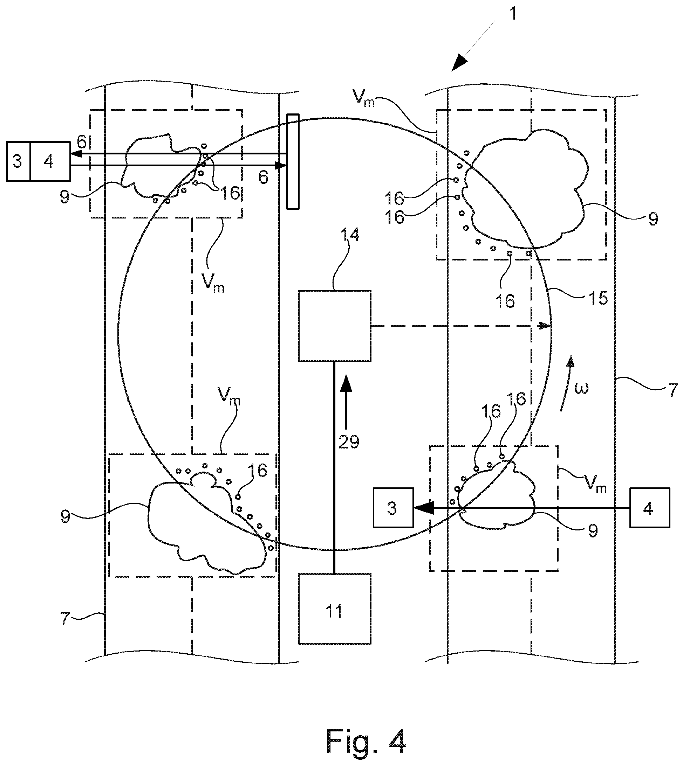

Fig. 3 zeigt eine vorteilhafte Ausführungsform der Messeinheit 4 mit Mehrfachmessung einer Abgaswolke 9. In dieser Ausführungsform sind Lichtquelle 2 und Detektor 3 in der Messeinheit 4 angeordnet. In einer Variante können die Lichtquelle 2 und/oder der Detektor 3 auch extern der Messeinheit 4 an einer beliebig geeigneten Stelle angeordnet sein. Die Bildeinheit 29 ist in Fig.3 nur angedeutet und kann wie oben im Zusammenhang mit Fig.2 erläutert oder auch wie im Zusammenhang mit Fig.4 unten erläutert ausgeführt sein. Die Bildeinheit 29 erzeugt die Abbildungen des Messvolumens $V_M$, die in der Auswerteeinheit 11 verarbeitet werden. In der Messeinheit 4 ist eine Multiplexereinheit 10 vorgesehen, welche eine Vielzahl von Lichtstrahlen 6 erzeugt (wobei in Fig.3 aus Gründen der Übersichtlichkeit nur einige mit Bezugszeichen bezeichnet sind). Die Multiplexereinheit 10 erzeugt dabei aus dem Primärlichtstrahl 17 (in Fig. 3 nicht eingezeichnet) der Lichtquelle 2 die Vielzahl von Lichtstrahlen 6.

Die Multiplexereinheit 10 kann beispielsweise durch einen "optical fiber multiplexer", "optical coupler" oder "optical splitter" realisiert werden. Auch eine Realisierung der Multiplexereinheit 10 als "Fiber Optic Switching Network" ist denkbar. Verschiedene Ausführungen einer Multiplexereinheit 10 sind hinreichend bekannt und der Fachmann kann eine für die jeweilige Anwendung geeignete wählen.

Die einzelnen, abgestrahlten Lichtstrahlen 6 werden durch verschiedene Stellen bzw. Bereiche des Messvolumens $V_M$ geleitet, womit die Lichtstrahlen 6 eine im Messvolumen $V_M$ befindliche Abgaswolke 9 auch an unterschiedlichen Stellen durchdringen. Damit kann die Abgaswolke 9 örtlich aufgelöst erfasst werden. Die einzelnen Lichtstrahlen 6 können von zumindest einen Detektor 3 erfasst werden oder an zumindest einer Spiegelungseinheit 8 (wie in Fig.3) wie oben beschrieben reflektiert und als reflektierte Lichtstrahlen 6 zu zumindest einem Detektor 3 gelenkt (der in der Ausführung der Fig.3 in der Messeinheit 4 angeordnet ist) und damit erfasst werden.

Natürlich kann für jeden Lichtstrahl 6 oder eine Gruppe von Lichtstrahlen 6 jeweils eine Spiegelungseinheit 8 vorgesehen sein. Genauso kann für jeden reflektierten Lichtstrahl 6 oder für eine Gruppe an reflektierten Lichtstrahlen 6 jeweils ein Detektor 3 vorgesehen sein.

Der zumindest eine Detektor 3 kann nun wie beschrieben eine Abnahme der Lichtintensität, bzw. eine Absorption A, für jeden erfassten Lichtstrahl 6 ermitteln und an die Auswerteeinheit 11 übermitteln. Genauso kann die Auswerteeinheit 11 aus der von der Bildeinheit 29 aufgenommenen zumindest einen Abbildung des Messvolumens $V_M$ für jeden Lichtstrahl 6, und gegebenenfalls zugehörigen reflektierten Lichtstrahl 6 (sofern dieser die Abgaswolke 9 durchdringt), wie beschrieben eine Gesamtdurchtrittsstrecke x ermitteln. Damit kann eine Konzentration c eines gasförmigen oder festen Stoffes an verschiedenen Stellen in der Abgaswolke 9 ermittelt werden.

Es ist offensichtlich, dass auch hier eine zweidimensionale örtliche Auflösung der Abgaswolke 9 möglich ist, wenn die Vorrichtung 1 so ausgeführt ist, dass zumindest zwei Gruppen von Lichtstrahlen 6 die Abgaswolke 9 in unterschiedlichen Richtungen (vorzugsweise normal aufeinander) durchdringen. Wenn eine solche zweidimensionale Auflösung mehrmals zeitlich hintereinander ausgeführt wird, kann daraus auch eine dreidimensionale Konzentrationsverteilung (bei einer bewegten Abgaswolke 9) oder ein zeitlicher Verlauf der Konzentrationsverteilung in einer Abgaswolke 9 ermittelt werden.

Die einzelnen Lichtstrahlen 6 sind im Ausführrungsbeispiel gemäß Fig. 3 in der Messeinheit 4 über optische Pfade 13 geführt, beispielsweise Lichtwellenleiter, an deren Enden die Lichtstrahlen 6 über je eine Auskoppeleinheit 12 abgestrahlt werden. Wenn zumindest zwei dieser optischen Pfade 13 mit unterschiedlichen optischen Weglängen ausgeführt sind, dann bewirkt das, dass die zu den optischen Pfaden mit unterschiedlichen Weglängen zugehörigen Rückstrahlen 6 aufgrund der unterschiedlichen Laufzeiten des Lichts zu unterschiedlichen Zeitpunkten an einem Detektor 3 ankommen. Das kann die Erfassung verschiedener Lichtstrahlen 6 mit einem einzelnen Detektor 3 vereinfachen oder erleichtern.

Es können in der Vorrichtung 1 natürlich auch eine Mehrzahl an Messeinheiten 4 mit Multiplexereinheiten 10, wie mit Bezugnahme auf die Fig.3 beschrieben, vorgesehen sein. Da ermöglicht wieder eine mehrfache Vermessung in einem Messvolumen $V_M$, vorzugsweise um eine zweidimensionale Auflösung des Messvolumens $V_M$ zu erzielen, oder auch die Messung unterschiedlicher Messvolumina $V_M$.

Eine ortsaufgelöste Ermittlung der Konzentration c in einer Abgaswolke 9 (beispielsweise mit einer Messeinheit nach Fig.3 oder mit mehreren Messeinheiten 4 wie in Fig.2 oder auch einer Kombination derartiger Anordnungen) kann dann beispielsweise genutzt werden, um eine kontinuierliche Verteilung eines gasförmigen oder festen Stoffes in der Abgaswolke 9 zu simulieren, beispielsweise datengetrieben oder mittels hybrider Modellierung. Unter hybrider Modellierung versteht man eine Kombination aus diskreten Termen, wie beispielsweise den gemessenen Konzentrationswerten, und kontinuierlichen Termen, beispielsweise "Fluid/gas dynamics" in einer Abgaswolke 9.

Fig. 4 zeigt eine weitere erfindungsgemäße Ausführungsform der Vorrichtung 1. In dieser Ausgestaltung sind mehrere Messeinheiten 4 (in Fig.4 sind aus Gründen der Übersichtlichkeit zwei davon angedeutet) angeordnet, die wie oben beschrieben ausgeführt sein können und für die jeweils ein Detektor 3 vorgesehen ist. Die mehreren Messeinheiten 4 werden verwendet, um in verschiedenen Messvolumina $V_M$ Konzentrationsmessungen eines gasförmigen oder festen Stoffes in einer Abgaswolke 9 im jeweiligen Messvolumen $V_M$ durchzuführen. Die Messvolumina $V_M$ können dabei unterschiedlich im Volumen sein, wobei die Volumina von der Messanordnung der Messeinheiten 4 abhängen. Die Strahlengänge zur Konzentrationsmessung sind in Fig. 4 aus Gründen der Übersichtlichkeit nicht vollständig eingezeichnet bzw. nur teilweise angedeutet. In der Ausgestaltung der Fig.4 ist eine Bildeinheit 29 vorgesehen, die von allen Abgaswolken 9 in den verschiedenen Messvolumina $V_M$ Abbildungen aufnimmt und an eine Auswerteeinheit 11 sendet. Die Auswerteeinheit 11 erhält natürlich auch Werte der mit Detektoren 3 ermittelten Absorptionen A.

Die Bildeinheit 29 ist in dieser Ausführung der Vorrichtung 1 als Lidareinheit 14 ausgeführt. Die Lidareinheit 14 kann örtlich in der Nähe der Messeinheiten 4 angeordnet sein, kann aber auch beispielsweise in einer bestimmten Höhe über den Messeinheiten 4 angeordnet sein, oder auch mittels Drohne mobil gesteuert werden, um eine Messkampagne durchzuführen.

Mit einer Lidareinheit 14 kann eine sehr genaue Vermessung einer Abgaswolke 9 durchgeführt werden, wobei die Lidareinheit 14 Abbildungen zumindest eines Teils der Abgaswolke 9 aufnimmt, um die Gesamtdurchtrittsstrecke x wie oben beschrieben zu ermitteln.

Die Lidareinheit 14 basiert auf einem Laser, beispielsweise einem YAG Laser mit 1064 nm oder 532 nm Wellenläge, oder ähnlichen Ausführungen, die der Fachmann für passend erachtet. Es können auch IR Laser verwendet werden, wobei jedoch eine hinreichende Abschirmung nötig sein kann, um Augenschäden zu vermeiden.

Eine Lidareinheit 14 im UV oder NIR Bereich kann aber beispielsweise auch genutzt werden, um gasförmige oder feste Stoffe direkt zu messen. Lidar wird unter anderem eingesetzt, um in atmosphärischen Messungen beispielsweise Kohlendioxid ($CO_2$), Schwefeldioxid ($SO_2$) und Methan ($CH_4$) nachzuweisen. Das kann benutzt werden, um beispielsweise grobe Abschätzungen von gasförmigen oder festen Stoffen in der vermessenen Abgaswolke 9 durchzuführen oder auch um eine zu der erfindungsgemäßen Konzentrationsmessung redundante Konzentrationsmessung zu erhalten.

Die Lidareinheit 14 kann sich entlang zumindest einer Achse bewegen bzw. um zumindest eine Achse verschwenkt werden, um das Laserlicht in verschiedene Richtungen auszusenden, so dass damit ein Abbild der Umgebung und einer gegebenenfalls vorhandenen Abgaswolke 9 aufgezeichnet werden kann. In der Ausführung nach Fig.4 wird die Lidareinheit 14 benutzt, um verschiedene Abgaswolken 9 in verschiedenen Messvolumina $V_M$ abzubilden. Dazu scannt die Lidareinheit 14 die Umgebung 15 (in Fig.4 durch einen Kreis rund um die Lidareinheit 14 dargestellt) und abhängig von der Reflexionszeit des ausgesendeten Laserimpulses werden Aufnahmen der Umgebung 15 gemacht. In Fig. 4 ist bespielhaft die Funktionsweise der Lidareinheit 14 in zwei Dimensionen dargestellt. Die Lidareinheit 14 rotiert um eine durch die Lidareinheit 14 normal auf die Zeichnungsebene von Fig. 4, verlaufende Achse und scannt in einer Kreisfrequenz $\omega$ die Umgebung 15. Dabei werden Abschnitte 16 der Abgaswolken 9 detektiert, zu Abbildungen der Abgaswolke 9 zusammengefügt und können somit von der Auswerteeinheit 11 zu einem Teil eines Abbilds der Abgaswolke 9 rekonstruiert werden.

Eine solche Lidareinheit 14 kann aber natürlich auch nur bei einem einzigen Messvolumen $V_M$, beispielsweise wie in Fig.2 oder 3, verwendet werden. Genauso kann eine Mehrzahl an Lidareinheiten 14 in der Vorrichtung 1 vorhanden sein, die eine genaueres und gegebenenfalls größeres Abbild der Abgaswolke 9 ermöglichen.

Es sind auch Kombinationen aus Lidareinheiten 14 und Kameras 5 denkbar. Damit können beispielsweise Konzentrationen gasförmiger Stoffe über die Lidareinheit 14 gemessen werden, während eine Konzentration eines festen Stoffes in der Abgaswolke 9 über eine erfindungsgemäße Messeinheit 4 erfasst werden kann. So kann eine repräsentative Konzentrationsmessung von mehreren kritischen gasförmigen und festen Stoffen der Abgaswolke 9 erfolgen.

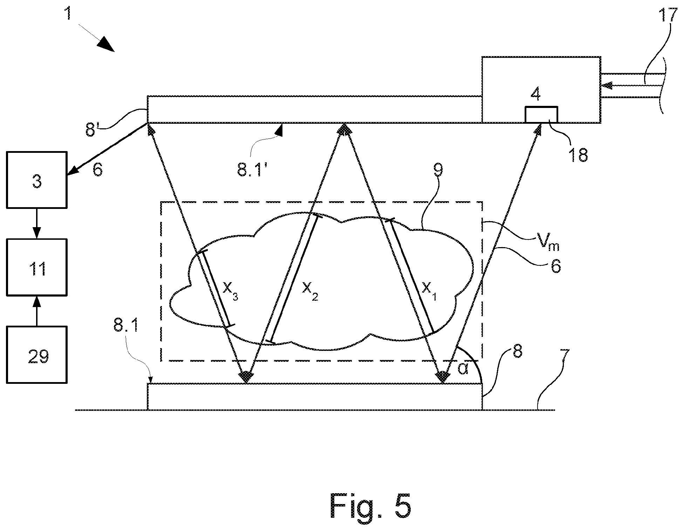

Fig. 5 zeigt eine weitere vorteilhafte Ausführungsform der Erfindung. In dieser Ausführungsform sind zwei voneinander beabstandet angeordnete Spiegelungseinheiten 8, 8' vorgesehen. Die jeweiligen Spiegelungsebenen 8.1, 8.1' der beiden Spiegelungseinheiten 8, 8' sind einander zugewandt angeordnet. Der Lichtstrahl 6 wird nun von der Messeinheit 4 in einem Winkel $\alpha$ abgestrahlt, der von einer Normalen auf die erste Spiegelungsebene 8.1 der ersten Spiegelungseinheit 8 abweicht. Der Lichtstrahl 6 wird an der ersten Spiegelungsebene 8.1 reflektiert und zur zweiten Spiegelungsebene 8.1' der zweiten Spiegelungseinheit 8' gelenkt. An dieser erfolgt eine weitere Reflexion des Lichtstrahls 6 zurück zu ersten Spiegelungseinheit 8. Der Lichtstrahl 6 läuft damit in einer Mehrzahl von Reflexionen zwischen der ersten Spiegelungseinheit 8 und der zweiten Spiegelungseinheit 8' hin und her und bewegt sich entlang einer Längserstreckung der Spiegelungsebenen 8.1, 8.1'. Abhängig vom Winkel $\alpha$ kann somit eine Mehrzahl an Reflexionen erzielt werden. Nach der letzten Reflexion am Ende der Spiegelungseinheiten 8, 8' wird der Lichtstrahl 6 in der Ausführungsform gemäß Fig.5 zu einem Detektor 3 gelenkt, der den Lichtstrahl 6 erfasst.

Nach der letzten Reflexion am Ende der Spiegelungseinheiten 8, 8' kann der Lichtstrahl 6 aber auch in einer nicht dargestellten Ausführungsform umgelenkt werden, beispielsweise durch einen Umlenkspiegel, und in einer Anzahl von weiteren Reflexionen zwischen der ersten Spiegelungseinheit 8 und der zweiten Spiegelungseinheit 8' entlang einer Längserstreckung der Spiegelungsebenen 8.1, 8.1'zurücklaufen. In diesem Fall kann der Detektor 3 in der Messeinheit 4 angeordnet sein. Der Detektor 3 kann aber grundsätzlich an jeder geeigneten Stelle der Vorrichtung 1 angeordnet sein, die es erlaubt, den Lichtstrahl 6 nach einer Mehrzahl von Reflexionen des Lichts zwischen der ersten Spiegelungseinheit 8 und der zweiten Spiegelungseinheit 8' zu erfassen.

Aufgrund der mehrfachen Reflexionen kommt es zu einer Mehrzahl n von Durchtritten des Lichtstrahls 6 durch eine Abgaswolke 9 im Messvolumen $V_M$. Die sich daraus ergebenden Teildurchtrittsstrecken xn des Lichts durch die Abgaswolke 9 können wie oben beschrieben mit der Bildeinheit 29 bestimmt werden, woraus wiederum die Gesamtdurchtrittsstrecke x als Summe der Teildurchtrittsstrecken xn (hier gilt n=1, 2, 3) folgt. Das erlaubt wiederum die Konzentrationsbestimmung zumindest eines gasförmigen oder festen Stoffes in der Abgaswolke 9.

Durch die Mehrzahl n von Durchtritten des Lichtstrahls 6 durch die Abgaswolke 9 kann die Sensitivität der Konzentrationsmessung weiter erhöht werden und es können auch sehr geringe Konzentrationen eines gasförmigen oder festen Stoffes in der Abgaswolke 9 ermittelt werden.

Um die Mehrzahl n von Durchtritten des Lichtstrahls 6 durch die Abgaswolke 9 selektiv und bedarfsgerecht einstellen zu können, kann in der Messeinheit 4 auch eine Positionierungsoptikeinheit 18 vorgesehen sein, die es erlaubt den Winkel $\alpha$ des abgestrahlten Lichtstrahls 6 zu verstellen. Eine solche Positionierungsoptikeinheit 18 kann beispielsweise ein x-y Galvanometer oder auch ein verstellbarer Spiegel sein.

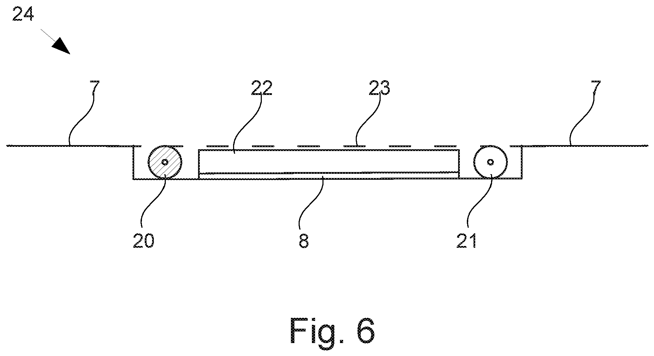

Eine weitere vorteilhafte Ausführungsform für einen Schutz einer Spiegelungseinheit 8 ist eine Schutzfolieneinheit 24, die in Fig.6 dargestellt ist. Diese dient dazu, eine Schutzfolie 23 austauschbar über einer Spiegelungseinheit 8 anzuordnen, um diese vor Verschmutzung oder Beschädigung (z.B. durch Kratzer) zu schützen. Die Schutzfolie 23 ist dabei hinreichend transparent ausgeführt. Eine verschmutzte Schutzfolie 23 kann bedarfsweise durch eine saubere Schutzfolie 23 ersetzt werden. Eine mögliche Ausführungsform einer Schutzfolieneinheit 24 besteht gemäß Fig.6 aus einer ersten Rolle 20, auf der saubere Schutzfolie 23 aufgewickelt ist. Von dieser ersten Rolle 20 kann saubere Schutzfolie 23 abgewickelt werden und über einer Spiegelungseinheit 8 angeordnet werden. Es kann eine zweite Rolle 21 vorgesehen sein, auf der die verschmutzte Schutzfolie 23 aufgewickelt werden kann. Bei bestimmungsgemäßer Verwendung wird bedarfsweise saubere Schutzfolie 23 von der ersten Rolle 20 abgewickelt und gleichzeitig verschmutzte Schutzfolie 23 von der zweiten Rolle 21 aufgewickelt. Die Spiegelungseinheit 8 ist in dieser vorteilhaften Ausführungsform unterhalb der Oberfläche 7 angeordnet.

Die abgewickelte Schutzfolie 23 ist dabei über der Spiegelungseinheit 8 angeordnet, um die Spiegelungseinheit 8 vor Verschmutzung oder Beschädigung zu schützen. Aus Stabilitätsgründen kann zwischen der Spiegelungseinheit 8 und der Schutzfolie 23 auch ein mechanischer Schutz 22 vorgesehen sein, welcher allerdings ausreichende optische Durchlässigkeit aufweisen sollte. Eine der beiden Rollen 20, 21 kann angetrieben sein, um ein bedarfsweises Weiterbewegen der Schutzfolie 23 über der Spiegelungseinheit 8 zu bewirken. Hierfür kann auch eine Automatisierungseinheit vorgesehen sein, die den Antrieb der angetriebenen Rolle 20, 21 ansteuert. Günstigerweise kann das Antreiben der Rollen 20, 21 bei Unterschreiten eines Grenzwerts erfolgen, beispielsweise einem Lichtintensitätsverlust eines mit einem Detektor 3 erfassten Lichtstrahls 6. Dann kann die Automatisierungseinheit die angetriebene Rolle 20, 21 automatisiert ansteuern, um die Schutzfolie 23 weiterzubewegen. Damit kann die verschmutzte Schutzfolie 23 oberhalb der Spiegelungseinheit 8 einfach und bedarfsweise durch eine unverschmutzte ausgetauscht werden. Das kann vorteilhaft sein, wenn die Spiegelungseinheit 8 generell hoher Verschmutzung ausgesetzt ist.

In der Schutzfolieneinheit 24 kann auch eine Heizvorrichtung, beispielsweise eine elektrische Heizung, integriert sein, vorzugsweise im mechanischen Schutz 22. Durch die Heizvorrichtung kann verhindert werden, dass bei Nässe, wie z.B. bei Regen, Nebel, Schnee, das optische Systeme der Vorrichtung 1 durch die Bildung von Eis oder Pfützen auf der Oberfläche der Schutzfolie 23 beeinträchtigt wird.

## Patentansprüche

1.  Vorrichtung zur Konzentrationsbestimmung zumindest eines gasförmigen oder festen Stoffes in zumindest einem Messvolumen ($V_M$) an einer stationären Messstelle, wobei zumindest eine Messeinheit (4) vorgesehen ist, einen Lichtstrahl (6) mit vorgegebener Lichtintensität (I) durch das Messvolumen ($V_M$) abzustrahlen, und zumindest ein Detektor (3) vorgesehen ist, um den Lichtstrahl (6) nach dem Durchtritt durch das Messvolumen ($V_M$) zu erfassen, wobei der zumindest eine Detektor (3) ausgebildet ist, eine Abnahme der Lichtintensität aufgrund des zumindest einen gasförmigen oder festen Stoffes im Messvolumen ($V_M$) zu ermitteln, wobei in der Vorrichtung (1) eine Bildeinheit (29) vorgesehen ist, um bei teilweisem oder vollständigem Vorhandensein einer Abgaswolke (9) im Messvolumen ($V_M$) zumindest einen Teil der Abgaswolke (9) aufzunehmen, wobei in der Vorrichtung (1) eine Auswerteeinheit (11) vorgesehen ist, die dazu eingerichtet ist, aus zumindest einer aufgenommenen Abbildung des zumindest einen Teils der Abgaswolke (9) eine Gesamtdurchtrittsstrecke (x) des Lichtstrahls (6) durch die Abgaswolke (9) im Messvolumen ($V_M$) zu bestimmen, und wobei die Auswerteeinheit (11) ferner dazu eingerichtet ist, aus der ermittelten Abnahme der Lichtintensität und der Gesamtdurchtrittsstrecke (x) eine Konzentration des gasförmigen oder festen Stoffes im Messvolumen ($V_M$) zu ermitteln.

2.  Vorrichtung nach Anspruch 1, **dadurch gekennzeichnet, dass** die zumindest eine Messeinheit (4) beabstandet von zumindest einer ersten Spiegelungseinheit (8) angeordnet ist, wobei das Messvolumen ($V_M$) zwischen der zumindest einen Messeinheit (4) und der zumindest einen ersten Spiegelungseinheit (8) ausgebildet ist, und die zumindest eine Messeinheit (4) dazu eingerichtet ist, einen Lichtstrahl (6) in Richtung der zumindest einen Spiegelungseinheit (8) abzustrahlen, und dass die zumindest eine erste Spiegelungseinheit (8) vorgesehen ist, den Lichtstrahl (6) nach Durchtritt durch das Messvolumen ($V_M$) zu reflektieren und zu zumindest einem Detektor (3) zu senden.

3.  Vorrichtung nach Anspruch 2, **dadurch gekennzeichnet, dass** der an der Spiegelungseinheit (8) reflektierte Lichtstrahl (6) das Messvolumen ($V_M$) vor Erreichen des zumindest einen Detektors (3) zumindest ein weiteres Mal durchdringt.

4.  Vorrichtung nach Anspruch 3, **dadurch gekennzeichnet, dass** die Gesamtdurchtrittsstrecke (x) die Summe der Teildurchtrittsstrecken aller Durchtritte des Lichtstrahls (6) durch die Abgaswolke (9) ist.

5.  Vorrichtung nach einem der Ansprüche 1 bis 4, **dadurch gekennzeichnet, dass** in der Messeinheit (4) eine Multiplexereinheit (10) vorgesehen ist, die eine Mehrzahl an Lichtstrahlen (6) erzeugt, und die Messeinheit (4) die Mehrzahl an Lichtstrahlen (6) an unterschiedlichen Stellen durch das Messvolumen ($V_M$) abstrahlt und zumindest ein Detektor (3) jeden der Mehrzahl an Lichtstrahlen (6), vorzugsweise nach einer Reflexion an einer Spiegelungseinheit (8), erfasst.

6.  Vorrichtung nach Anspruch 5, **dadurch gekennzeichnet, dass** zumindest zwei der Mehrzahl an Lichtstrahlen (6) in der Messeinheit (4) über jeweils einen optischen Pfad (13) geführt sind, wobei vorzugsweise die optischen Pfade (13) zumindest teilweise unterschiedliche optische Weglängen aufweisen.

7. Vorrichtung nach Anspruch 5 oder 6, **dadurch gekennzeichnet, dass** die Messeinheit (4) zumindest einen ersten Lichtstrahl (6) der Mehrzahl an Lichtstrahlen (6) in einer ersten Richtung durch das Messvolumen ($V_M$) abstrahlt und die Messeinheit (4) einen zweiten Lichtstrahl (6) der Mehrzahl an Lichtstrahlen (6) in einer zweiten Richtung durch das Messvolumen ($V_M$) abstrahlt, wobei die erste Richtung unterschiedlich zur zweiten Richtung ist.

8. Vorrichtung nach einem der Ansprüche 2 bis 7, **dadurch gekennzeichnet, dass** in der Vorrichtung eine zweite Spiegelungseinheit (8') vorgesehen ist, die beabstandet und gegenüberliegend von der zumindest einen ersten Spiegelungseinheit (8) angeordnet ist und die Messeinheit (4) den Lichtstrahl (6) in einem von einer Normalen auf eine erste Spiegelungsebene (8.1) der ersten Spiegelungseinheit (8) abweichenden Winkel abstrahlt, wobei die erste Spiegelungseinheit (8) den von der Messeinheit (4) abgestrahlten Lichtstrahl (6) zur gegenüberliegenden zweiten Spiegelungseinheit (8') reflektiert und die gegenüberliegende zweite Spiegelungseinheit (8') den Lichtstrahl (6) wieder zur ersten Spiegelungseinheit (8) zurückreflektiert, wobei der zumindest eine Detektor (5) dazu eingerichtet ist, den Lichtstrahl (6) nach einer Mehrzahl solcher Reflexionen zu erfassen.

9. Vorrichtung nach Anspruch 8, **dadurch gekennzeichnet, dass** in der Messeinheit (4) eine Positionierungsoptikeinheit (18) vorgesehen ist, mit der der Winkel des Lichtstrahls (6) verstellbar ist.

10. Vorrichtung nach einem der Ansprüche 1 bis 9, **dadurch gekennzeichnet, dass** in der Vorrichtung (1) mehrere Messeinheiten (4) vorgesehen sind, wobei zumindest zwei der Messeinheiten (4) einen Lichtstrahl (6) durch unterschiedliche Messvolumina ($V_M$) abstrahlen.

11. Vorrichtung nach einem der Ansprüche 1 bis 10, **dadurch gekennzeichnet, dass** in der Vorrichtung (1) mehrere Messeinheiten (4) vorgesehen sind, wobei zumindest zwei der Messeinheiten (4) einen Lichtstrahl (6) mit unterschiedlichen Richtungen durch dasselbe Messvolumen ($V_M$) abstrahlen.

12. Vorrichtung nach einem der Ansprüche 1 bis 11, **dadurch gekennzeichnet, dass** die Bildeinheit (29) zumindest eine Kamera (5) und/oder zumindest eine Lidareinheit (14) aufweist.

13. Vorrichtung nach einem der Ansprüche 2 bis 12, **dadurch gekennzeichnet, dass** eine Schutzfolie (23) austauschbar über der zumindest einen Spiegelungseinheit (8) angeordnet ist.

14. Verfahren zur Konzentrationsbestimmung (1) zumindest eines gasförmigen oder festen Stoffes in einer Abgaswolke (9) in zumindest einem Messvolumen ($V_M$) an einer stationären Messstelle, wobei zumindest eine Messeinheit (4) einen Lichtstrahl (6) mit einer vorgegebenen Lichtintensität (I) durch die Abgaswolke (9) im Messvolumen ($V_M$) abstrahlt, der Lichtstrahl (6) nach Durchtritt durch die Abgaswolke (9) von einem Detektor (3) erfasst wird und der Detektor (3) eine Abnahme der Lichtintensität des Lichtstrahls (6) aufgrund des zumindest einen gasförmigen oder festen Stoffes ermittelt, wobei zumindest ein Teil der Abgaswolke (9) im Messvolumen ($V_M$) von einer Bildeinheit (29) aufgenommen wird und aus der aufgenommenen Abbildung des zumindest einen Teils der Abgaswolke (9) eine Gesamtdurchtrittsstrecke (x) des Lichtstrahls (6) durch die Abgaswolke (9) im Messvolumen ($V_M$) bestimmt wird, **und** wobei aus der ermittelten Abnahme der Lichtintensität und der Gesamtdurchtrittsstrecke (x) eine Konzentration des gasförmigen oder festen Stoffes im Messvolumen ($V_M$) ermittelt wird.

**Claims**

1. A device for determining the concentration of at least one gaseous or solid material in at least one measurement volume ($V_M$) at a stationary measurement point, wherein at least one measurement unit (4) is provided to emit a light beam (6) with a predetermined light intensity (I) through the measurement volume ($V_M$), and at least one detector (3) is provided to detect the light beam (6) after it has passed through the measurement volume ($V_M$), wherein the at least one detector (3) is configured to determine a decrease in the light intensity due to the at least one gaseous or solid material in the measurement volume ($V_M$), whereas an image unit (29) is provided in the device (1), in order to capture at least a part of an exhaust plume (9) in the case of partial or complete presence of an exhaust plume (9) in the measurement volume ($V_M$), whereas an evaluation unit (11) is provided in the device (1) which is configured to determine a total passage path (x) of the light beam (6) through the exhaust plume (9) in the measurement volume ($V_M$) from at least one captured image of the at least one part of the exhaust plume (9), whereas the evaluation unit (11) is further configured to determine a concentration of the gaseous or solid material in the measurement volume ($V_M$) from the determined decrease in the light intensity and the total passage path (x).

2. The device according to claim 1, **characterized in that** the at least one measurement unit (4) is arranged at a distance from at least one first reflection unit (8), the measuring volume ($V_M$) being formed between the at least one measurement unit (4) and the at least one first reflection unit (8), and the at least one measurement unit (4) is configured to emit a light beam (6) in the direction of the at least one reflection unit (8), and **in that** the at least one first reflection unit (8) is provided to reflect the light beam (6) after it has passed through the measurement volume ($V_M$) and to transmit it to at least one detector (3).

3. The device according to claim 2, **characterized in that** the light beam (6) reflected at the reflection unit (8) passes through the measurement volume ($V_M$) at least once more before reaching the at least one detector (3).

4. The device according to claim 3, **characterized in that** the total passage path (x) is the sum of the partial passage paths of all passages of the light beam (6) through the exhaust plume (9).

5. The device according to any of claims 1 to 4, **characterized in that** a multiplexer unit (10) is provided in the measurement unit (4), which multiplexer unit generates a plurality of light beams (6), and the measurement unit (4) emits the plurality of light beams (6) at different locations through the measurement volume ($V_M$) and at least one detector (3) detects each of the plurality of light beams (6), preferably after a reflection at a reflection unit (8).

6. The device according to claim 5, **characterized in that** at least two of the plurality of light beams (6) are guided in the measurement unit (4) via an optical path (13) in each case, wherein the optical paths (13) preferably have different optical path lengths at least in part.

7. The device according to claim 5 or 6, **characterized in that** the measuring unit (4) emits at least one first light beam (6) of the plurality of light beams (6) in a first direction through the measuring volume ($V_M$) and the measuring unit (4) emits a second light beam (6) of the plurality of light beams (6) in a second direction through the measuring volume ($V_M$), the first direction being different from the second direction.

8. The device according to any of claims 2 to 7, **characterized in that** a second reflection unit (8') is provided in the device, which is arranged at a distance from and opposite the at least one first reflection unit (8) and the measurement unit (4) emits the light beam (6) at an angle deviating from a normal to a first reflection plane (8.1) of the first reflection unit (8), wherein the first reflection unit (8) reflects the light beam (6) emitted by the measurement unit (4) to the opposite second reflection unit (8') and the opposite second reflection unit (8') reflects the light beam (6) back to the first reflection unit (8), wherein the at least one detector (5) is set up to detect the light beam (6) after a plurality of such reflections.

9. The device according to claim 8, **characterized in that** a positioning optical unit (18) is provided in the measurement unit (4), with which the angle of the light beam (6) is adjustable.

10. The device according to any of claims 1 to 9, **characterized in that** in the device (1), a plurality of measurement units (4) is provided, wherein at least two of the measurement units (4) emit a light beam (6) through different measurement volumes ($V_M$).

11. The device according to any of claims 1 to 10, **characterized in that** a plurality of measurement units (4) are provided in the device (1), wherein at least two of the measurement units (4) emit a light beam (6) with different directions through the same measurement volume ($V_M$).

12. The device according to any of claims 1 to 11, **characterized in that** the image unit (29) comprises at least one camera (5) and/or at least one lidar unit (14).

13. The device according to any of claims 2 to 12, **characterized in that** a protective foil (23) is arranged replaceably above the at least one reflection unit (8).

14. A method for determining the concentration (1) of at least one gaseous or solid material in an exhaust plume (9) in at least one measuring volume ($V_M$) at a stationary measuring point, wherein at least one measuring unit (4) emits a light beam (6) with a predetermined light intensity (I) through the exhaust plume (9) in the measuring volume ($V_M$), the light beam (6) is detected by a detector (3) after passing through the exhaust plume (9) and the detector (3) determines a decrease in the light intensity of the light beam (6) due to the at least one gaseous or solid material, whereas at least a part of the exhaust plume (9) in the measuring volume ($V_M$) is captured by an imaging unit (29) and a total passage path (x) of the light beam (6) through the exhaust plume (9) in the measuring volume ($V_M$) is determined from the captured image of the at least one part of the exhaust plume (9), and whereas a concentration of the gaseous or solid material in the measuring volume ($V_M$) is determined from the determined decrease in light intensity and the total passage path (x).

## Revendications

1. Dispositif permettant la détermination de la concentration d'au moins une matière gazeuse ou solide dans au moins un volume de mesure ($V_M$) en un point de mesure stationnaire, dans lequel au moins une unité de mesure (4) est prévue pour émettre un faisceau lumineux (6) d'une intensité lumineuse (I) prédéfinie à travers le volume de mesure ($V_M$), et au moins un détecteur (3) est prévu pour détecter le faisceau lumineux (6) après son passage à travers le volume de mesure ($V_M$), dans lequel l'au moins un détecteur (3) est conçu pour déterminer une diminution de l'intensité lumineuse en raison de l'au moins une matière gazeuse ou solide dans le volume de mesure ($V_M$), dans lequel une unité d'imagerie (29) est prévue dans le dispositif (1) pour enregistrer au moins une partie du nuage de gaz d'échappement (9) en cas de présence partielle ou totale d'un nuage de gaz d'échappement (9) dans le volume de mesure ($V_M$), dans lequel une unité d'évaluation (11) est prévue dans le dispositif (1), laquelle est conçue pour

   définir, à partir d'au moins une image enregistrée de l'au moins une partie du nuage de gaz d'échappement (9), une distance totale de passage (x) du faisceau lumineux (6) à travers le nuage de gaz d'échappement (9) dans le volume de mesure ($V_M$), et dans lequel l'unité d'évaluation (11) est en outre conçue pour déterminer, à partir de la diminution déterminée de l'intensité lumineuse et de la distance totale de passage (x), une concentration de la matière gazeuse ou solide dans le volume de mesure ($V_M$).

2. Dispositif selon la revendication 1, **caractérisé en ce que** l'au moins une unité de mesure (4) est disposée à distance d'au moins une première unité de réflexion (8), dans lequel le volume de mesure ($V_M$) est formé entre l'au moins une unité de mesure (4) et l'au moins une première unité de réflexion (8), et l'au moins une unité de mesure (4) est conçue pour émettre un faisceau lumineux (6) en direction de l'au moins une unité de réflexion (8), et **en ce que** l'au moins une première unité de réflexion (8) est prévue pour réfléchir le faisceau lumineux (6) après son passage à travers le volume de mesure ($V_M$) et pour l'envoyer à au moins un détecteur (3).

3. Dispositif selon la revendication 2, **caractérisé en ce que** le faisceau lumineux (6) réfléchi par l'unité de réflexion (8) traverse au moins une autre fois le volume de mesure ($V_M$) avant d'atteindre l'au moins un détecteur (3).

4. Dispositif selon la revendication 3, **caractérisé en ce que** la distance de passage totale (x) est la somme des distances de passage partielles de tous les passages du faisceau lumineux (6) à travers le nuage de gaz d'échappement (9).

5. Dispositif selon l'une des revendications 1 à 4, **caractérisé en ce qu'**une unité de multiplexage (10) est prévue dans l'unité de mesure (4), laquelle génère une pluralité de faisceaux lumineux (6), et l'unité de mesure (4) émet la pluralité de faisceaux lumineux (6) à différents endroits à travers le volume de mesure ($V_M$) et au moins un détecteur (3) capte chacun de la pluralité de faisceaux lumineux (6), de préférence après une réflexion sur une unité de réflexion (8).

6. Dispositif selon la revendication 5, **caractérisé en ce qu'**au moins deux de la pluralité de faisceaux lumineux (6) dans l'unité de mesure (4) sont guidés respectivement par l'intermédiaire d'un chemin optique (13), dans lequel les chemins optiques (13) présentent de préférence au moins partiellement des longueurs de trajet optique différentes.

7. Dispositif selon la revendication 5 ou 6, **caractérisé en ce que** l'unité de mesure (4) émet au moins un premier faisceau lumineux (6) de la pluralité de faisceaux lumineux (6) dans une première direction à travers le volume de mesure ($V_M$) et l'unité de mesure (4) émet un deuxième faisceau lumineux (6) de la pluralité de faisceaux lumineux (6) dans une deuxième direction à travers le volume de mesure ($V_M$), la première direction étant différente de la deuxième direction.

8. Dispositif selon l'une des revendications 2 à 7, **caractérisé en ce qu'**il est prévu dans le dispositif une seconde unité de réflexion (8') qui est disposée à distance de et opposée à l'au moins une première unité de réflexion (8) et l'unité de mesure (4) émet le faisceau lumineux (6) selon un angle différent d'une normale à un premier plan de réflexion (8.1) de la première unité de réflexion (8), dans lequel la première unité de réflexion (8) réfléchit le faisceau lumineux (6) émis par l'unité de mesure (4) vers la seconde unité de réflexion (8') opposée et la seconde unité de réflexion (8') opposée réfléchit à nouveau le faisceau lumineux (6) vers la première unité de réflexion (8), dans lequel l'au moins un détecteur (5) est conçu pour détecter le faisceau lumineux (6) après une pluralité de telles réflexions.

9. Dispositif selon la revendication 8, **caractérisé en ce qu'**une unité optique de positionnement (18) est prévue dans l'unité de mesure (4), avec laquelle l'angle du faisceau lumineux (6) peut être réglé.

10. Dispositif selon l'une des revendications 1 à 9, **caractérisé en ce que** plusieurs unités de mesure (4) sont prévues dans le dispositif (1), dans lequel au moins deux des unités de mesure (4) émettent un faisceau lumineux (6) à travers différents volumes de mesure ($V_M$).

11. Dispositif selon l'une des revendications 1 à 10, **caractérisé en ce que** plusieurs unités de mesure (4) sont prévues dans le dispositif (1), dans lequel au moins deux des unités de mesure (4) émettent un faisceau lumineux (6) avec des directions différentes à travers le même volume de mesure ($V_M$).

12. Dispositif selon l'une des revendications 1 à 11, **caractérisé en ce que** l'unité d'imagerie (29) présente au moins une caméra (5) et/ou au moins une unité lidar (14).

13. Dispositif selon l'une des revendications 2 à 12, **caractérisé en ce qu'**un film de protection (23) est disposé de manière interchangeable sur l'au moins une unité de réflexion (8).

14. Procédé permettant la détermination de la concentration (1) d'au moins une matière gazeuse ou solide dans un nuage de gaz d'échappement (9) dans au moins un volume de mesure ($V_M$) à un point de mesure stationnaire, dans lequel au moins une unité de mesure (4) émet un faisceau lumineux (6) avec une intensité lumineuse (I) prédéfinie à travers le nuage de gaz d'échappement (9) dans le volume de mesure ($V_M$), le faisceau lumineux (6) est détecté par un détecteur (3) après son passage à travers le nuage de gaz d'échappement (9) et le détecteur (3) détermine une diminution de l'intensité lumineuse du faisceau lumineux (6) en raison de l'au moins une matière gazeuse ou solide, dans lequel au moins une partie du nuage de gaz d'échappement (9) est enregistrée dans le volume de mesure ($V_M$) par une unité d'imagerie (29) et une distance totale de passage (x) du faisceau lumineux (6) à travers le nuage de gaz d'échappement (9) dans le volume de mesure ($V_M$) étant définie à partir de l'image enregistrée d'au moins une partie du nuage de gaz d'échappement (9), et dans lequel une concentration de la matière gazeuse ou solide dans le volume de mesure ($V_M$) est déterminée à partir de la diminution déterminée de l'intensité lumineuse et de la distance totale de passage (x).

Fig. 1

Fig. 2

Fig. 3

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4924095 A
- CN 103712929 A1
- EP 3789755 A